# EUROPEAN PATENT APPLICATION

(11) **EP 1 447 085 A1**
(43) Date of publication of application: **18.08.2004**
(21) Application number: 02801553.5
(22) Date of filing: 11.10.2002
(51) Int. Cl.: A61K 31/70, A61K 33/06, A61K 45/00, A61P 19/00, A61P 43/00, A23L 1/30

(54) **AGENT CONTROLLING THE PRODUCTION OF OSTEOCLAST FORMATION REGULATOR AND METHOD OF SCREENING SUBSTANCE CAPABLE OF CONTROLLING THE PRODUCTION OF OSTEOCLAST FORMATION REGULATOR**

(30) Priority: 15.10.2001 JP 2001317358
(71) Applicant: Kabushiki Kaisha Hayashibara Seibutsu Kagaku Kenkyujo, Okayama-shi, Okayama 700-0907 (JP)
(72) Inventor: ARIYASU, T. K.K. Hayashibara Seibutsu Kagaku Kenk., Okayama-shi, Okayama 700--0907 (JP); HANAYA, T. K. K. Hayashibara Seibutsu Kagaku Kenk., Okayama-shi, Okayama 700-0907 (JP); ARAI, S. K. K. Hayashibara Seibutsu Kenkyujo, Okayama-shi, Okayama 700-0907 (JP); IKEDA, M. K. K. Hayashibara Seibutsu Kagaku Kenk., Okayama-shi, Okayama 700-0907 (JP); KURIMOTO, M. K.K. Hayashibara Seibutsu Kagaku Kenk, Okayama-shi, Okayama 700-0907 (JP)
(74) Representative: Daniels, Jeffrey Nicholas
(86) International application number: PCT/JP2002/010604
(87) International publication number: WO 2003/033003

(57) **Abstract**

The present invention has the object to provide an agent for controlling the production of osteoprotegerin (OPG) and a method for screening substances capable of controlling the production of OPG, and it solves the object by providing the agent for controlling the production of OPG, which comprises a saccharide as an effective ingredient; and a method for screening a substance capable of controlling the production of OPG, which comprises the steps of culturing an established cell line capable of producing OPG in a nutrient culture medium; adding a test sample to the cell culture; culturing the cells in the presence of the test sample to produce OPG; assaying the production level of OPG in the culture supernatant; and judging the test sample to be a positive substance that can control the production of OPG when the production level of OPG for the test sample is higher or lower than that for a control system.

## Description

### Technical Field

The present invention relates to a novel agent for controlling the production of osteoclast-formation (or osteoclastogenesis) inhibitory factor, more particularly, an agent for controlling the production of osteoclastogenesis inhibitory factor, which comprises a saccharide as an effective ingredient, and a method for screening substances capable of controlling the production of osteoclastogenesis inhibitory factor.

### Background Art

Osteoclastogenesis inhibitory factor also known as osteoprotegerin (abbreviated as "OPG" hereinafter) is known to be produced from osteoblasts and organs such as liver, kidney, lung, and heart of mammals and humans, and it has been highlighted in these days as an important bone resorption inhibitor. Simonet WS et al. reported the gene for coding OPG in Cell, Vol. 89, pp. 309-319 (1997), and this accelerated the study of OPG rapidly. It is said that OPG exists in blood, acts on the bone metabolism systemically, and inhibits the formation of osteoclast. Thus, as a means for normalizing mammals and humans from abnormal osteoclast formation conditions, if the production level of OPG in mammals in such abnormal conditions could be controlled, the formation of osteoclast in abnormal conditions would be improved and maintained in normal conditions, and the bone would be normalized and kept in healthy conditions.

However, there has been found almost no report on controlling the production of OPG. Under these circumstances, required is the establishment of agents which may control the production of OPG and which can be used safely, easily, and daily at users' homes without restricting to their use in hospitals and clinics.

In view of these backgrounds, the first object of the present invention is to establish an agent for controlling the production of OPG which controls the production of OPG and which can be used safely, easily, and daily at users' homes without restricting to their use in hospitals and clinics. The second object of the present invention is to provide a method for screening substances, which can be incorporated into such agent as an effective ingredient, promptly, easily, and accurately.

### Disclosure of Invention

During energetically studying the physiological functions of saccharides, particularly, α,α-trehalose, α,β-trehalose, and β,β-trehalose as non-reducing saccharides (these trehalose isomers are designated as "trehalose" throughout the specification, unless specified otherwise), the present inventors discovered that the culture of epithelial cells of human small intestinal mucosae in the presence of trehalose improved the production level of OPG, and that, among different saccharides, there exist some saccharides which inhibit the production of OPG. Also, the present inventors discovered that culturing of an established cell line, which produces OPG in the presence of trehalose, increased the production level of OPG in the culture. Based on these findings, the present invention was made.

The present invention solves the above first object by providing an agent for controlling the production of OPG, which comprises a substance capable of controlling the production of OPG.

The present invention solves the above second object by providing a method for screening a substance capable of controlling the production of OPG.

### Brief Explanation of Accompanying Drawing

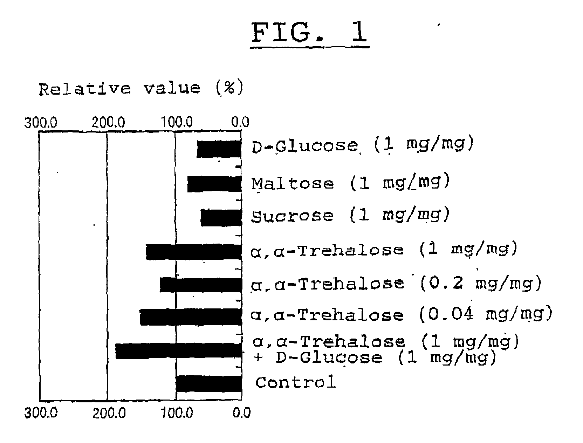

FIG. 1 is a figure for the relative expression intensity of OPG contained in each supernatant after 2-day cell culture in the presence or the absence of different saccharides.

### Best Mode for Carrying out the Invention

The present invention relates to an agent for controlling the production of OPG, which comprises a saccharide as an effective ingredient, and to a method for screening substances capable of controlling the production of OPG. The saccharides usable in the present invention include one or more of the following saccharides alone or in an appropriate combination: Monosaccharides such as D-glucose, D-mannose, D-galactose, D-arose, D-altrose, D-idose, D-arabinose, D-ribose, D-xylose, D-lyxose, D-fucose, D-fructose, D-talose, L-sorbose, D-tagatose, and D-psicose; disaccharides such as maltose, trehalose, sucrose, lactose, turanose, and rutinose; oligosaccharides of trisaccharides or higher such as maltotriose and lactosucrose; and cyclic saccharides such as tetrasaccharide having the structure of cyclo{→6)-α-D-glucopyranosyl-(1→3)-α-D-glucopyranosyl-(1→6)-α-D-glucopyranosyl-(1→3)-α-D-glucopyranosyl-(1→}, and cyclodextrins. In the present invention, among these saccharides, trehalose is particularly suitably used as a substance, which controls the production of OPG in mammals and humans, i.e., as a substance which enhances the production of OPG. In the case of using trehalose as an effective ingredient for the agent of the present invention, one or more of isomers of trehalose, i.e., α,α-trehalose, α,β-trehalose, and β,β-trehalose can be arbitrarily used in an effective amount. These isomers should not be restricted to homogeneous ones, and any trehalose can be used independently of their preparation methods, purities, properties, and origins as long as other ingredients, coexisted with the isomers, do not hinder the action of trehalose.

As regards the process for producing trehalose usable in the present invention, detailed description thereof is omitted because the present invention does not relate to such a process in itself. However, when the feasibility, production cost, and property of trehalose to be produced are weighed, preferably employed are the processes comprising a step of subjecting partial starch hydrolyzates to the actions of non-reducing saccharide-forming enzyme and trehalose-releasing enzyme, disclosed in Japanese Patent Kokai Nos. 143,876/95, 213,283/95, 322,883/95, 298,880/95, 66,187/96, 66,188/96, 336,388/96, and 84,586/96, applied for by the same applicant as the present invention. With these processes, a high quality trehalose can be easily produced in a higher yield from starches as a relatively cheap material. Examples of Commercialized trehalose products prepared by such processes are "TREHA® ", a crystalline trehalose powder having a trehalose content of 98% or more, commercialized by Hayashibara Shoji, Inc., Okayama, Japan; and "TREHASTAR®", a trehalose syrup having a trehalose content of 28% or more, commercialized by Hayashibara Shoji, Inc., Okayama, Japan. α,α-Trehalose can be also produced by subjecting maltose either to the action of a maltose/trehalose converting enzyme disclosed in Japanese Patent Kokai Nos. 170,977/95, 263/96, or 149,980/96, applied for by the same applicant as the present invention; or to the coaction of conventional maltose phosphorylase and trehalose phosphorylase.

To produce α,β-trehalose, cyolomatodextrin glucanotransferase (CGTase), and β-galactosidase can be allowed to act in this order on a mixture of lactose and partial starch hydrolyzate, according to the methods disclosed in Japanese Patent Kokai Nos. 144,694/92 and 179,490/92, applied for by the same applicant as the present invention. While, β,β-trehalose can be produced by conventional chemical syntheses. Since the agent for controlling the production of OPG according to the present invention is orally or intubationally administered to intestinal tracts of subjects, the saccharides used for such purposes should not be restricted to those with the highest possible purity and also include those with a relatively low purity, i.e., those which comprise the desired saccharides and other ingredients that are unavoidably formed as by-products, depending or their preparation methods; those in a mixture form of the desired saccharides and other appropriate ingredients which do not substantially hinder the action of the saccharides for controlling the production of OPG in mammals and humans.

The substances, which control the production of OPG, usable in the present invention include as a whole those which control the production of OPG and can be selected by the later described method for screening such substances. In the present invention, saccharides are exemplified as such substances screened by the above-mentioned method, however, any substance can be also used similarly as the above saccharides as long as they are capable of controlling the production of OPG and orally or intubationally administered to mammals and humans. Accordingly, one or more substances, that are selected by the screening method of the present invention, can be used as the effective ingredients for the agent for controlling the production of OPG of the present invention.

The agent for controlling the production of OPG according to the present invention can be in a single form of a substance capable of controlling the production of OPG, or in a composition form comprising such a substance and other ingredient(s). Usually, the agent of the present invention is provided in the form of a composition such as a food, health food, health supplement, or pharmaceutical, which can be orally administered or intubationally administered to subjects in the form of a tube fed liquid diet or intubation clysis; a composition in the form of a solution, suspension, emulsion, cream, paste, powder, granule, or other shaped-product with the desired form. When the composition is in the form of a food product, materials and/or ingredients, which are generally used in food products, such as water, alcohols, amylaceous substances, proteins, fibers, saccharides, lipids, vitamins, minerals, flavors, colors, sweeteners, seasonings, stabilizers, antioxidants, antiseptics, viscosity-imparting agents, and fillers can be incorporated into the composition to ease the intake of the substances capable of controlling the production of OPG. To the above-identified compositions, one or more of other materials for health supplements such as bifid bacteria, saccharides for promoting the growth of bifid bacteria, milk powders, hydrolyzates of milk protein including casein calcium peptides and Casein phosphopeptides, lactoferrin, soybean isoflavone, blood meal, bone meal, shellfish meal, and coral meal. These food products may be in an orally administrable form or an intubationally administrable form such as those of tube fed liquid diets and intubation clyses.

In case the composition is in the form of a pharmaceutical, one or more of carriers, excipients/adjuvants, diluents, and stabilizers are mixed with other ingredients, and optionally further mixed with one or more of calcium agents such as calcium lactate, calcium glycerophosphate, calcium hydrogenphosphate, and calcium L-asparaginate, and other medicaments such as analgesics, anti-inflamatories, active vitamin D, vitamin K preparations, calcitonin preparations, estrogen preparations, and anabolic hormone preparations. Varying depending on actual use, the agent of the present invention usually contains at least 0.1% (w/w) ("% (w/w)" is abbreviated as "%" hereinafter), preferably, at least 1% of a substance(s) capable of controlling the production of OPG.

Now explaining the use of the agent of the present invention for humans, the agent is used to act on epithelial cells of human small intestinal mucosae and to effectively control the production of OPG in the epithelia cells, independently of its oral or incubational administration. Also, the agent of the present invention acts on epithelial cells of mammals other than human small intestinal mucosae, controls the production of OPG in the epithelia cells, and effectively inhibits the formation of osteoclast. Based on these, the agent of the present invention can be advantageously used as an agent for controlling the production of OPG and also used to prevent, treat, or improve bone-related diseases such as bone fractures and cracks. Varying depending on the intended use, in the case of improving/promoting the health conditions of bone, the agent of the present invention is orally administered to subjects usually in the form of a food product. For the purpose of relieving low-back-pain and arthralgia and improving the therapeutic effect of the agent against bone damages/diseases, the agent is orally administered to subjects in the form of, for example, a food product or a pharmaceutical such as a liquid, syrup, powder, granule, tablet, or capsule; or intubationally administered to subjects in the form of a liquid. The dose of the agent of the present invention is administered to subjects at a dose, usually, of about 1 to 100 g/adult/day, preferably, about 2 to 50 g/adult/day, daily or at a frequency of one to five shots per week. Varying depending on the purpose of the agent to be used and the symptoms of humans or mammals to be administered, the administration term is usually at least one month, preferably, at least three months, more preferably, at least six months. When saccharides are used as the effective ingredient of the agent of the present invention, they are most preferably applicable to practicing the present invention because such saccharides are effectively used as energy sources for living bodies and administrable for a relatively long period of time.

The method for screening substances, which control the production of OPG according to the present invention, is explained below: The method comprises the steps of culturing an established cell line capable of controlling the production of OPG in a nutrient culture medium, adding a test sample to the cell culture, culturing the cell line in the presence of the test sample, assaying the production level of OPG released in the culture supernatant, comparing the production level of OPG with that of control, and judging the test sample to be a positive substance capable of controlling the production of OPG when the production level of OPG for the test sample is higher or lower than that for a control system. The screening method has an advantage that it screens targeted substances which control the production of OPG, promptly, easily, and accurately.

Preferable examples of such established cell lines are MG-63 cells, ATCC CRL-1427, an established cell line derived from human osteosarcoma; and FHs 74 Int cells, ATCC CCL-241, an established cell line derived from a human fetus small intestine. In practicing the present invention, the established cell lines usable in the present invention should not be restricted to the above-identified cell lines, and any cell lines can be used as long as they produce OPG. As the nutrient culture media used in culturing such cell lines, those free of saccharide are preferably used; MEM and RPMI 1640 media, which are free of saccharide and serum and in which the above cells can grow, are preferably used. In culturing the above cells, they are suspended in a culture medium to give a final cell concentration of 1 x 10³ to 1 x 10⁷ cells/ml, preferably, 5 x 10³ to 1 x 10⁶ cells/ml, distributed to culture vessels having at least one or more wells, for example, those in the form of a plate having at least one or more wells, cultured at 30 to 40° C, preferably, 35 to 38° C for 24 to 72 hours in a 5% (v/v) CO₂ incubator, and washed several times with a fresh preparation of the same medium as used in the culture, followed by removing each supernatant from the wells. To the remaining cells in each well was added a prescribed volume of a medium prepared by stepwisely diluting a test sample with a fresh preparation of the same medium as used in the culture, and cultured for one to five days under the same conditions as in the above culture. As a control, a system free of test sample was used. At a prescribed timing during cell culture, a portion of each culture medium and that for control were respectively sampled, followed by assaying the production level of OPG for each sample, comparing the production level of OPG for the test sample with that of control, and judging the test sample as a positive substance which can control the production of OPG when the level of OPG for the test sample is higher or lower than that for the control system.

The method for screening substances, which can control the production of OPG, according to the present invention has a feature of screening substances with such potential easily, effectively, and promptly. Also the method has advantageous features that it does not need any experimental animal and has beneficial convenience and satisfactory cost-performance in such a manner of being easily practiced even in a relatively small experimental/clinical laboratories.

The efficacy and the safety of the agent of the present invention is explained with reference to the following experiments:

### Experiment 1: Production control of OPG by substance capable of controlling the production of OPG

### Experiment 1-1: Preparation of cell culture

FHs 74 Int cells, ATCC CCL 241, were inoculated to 96-well plates, with D-MEM medium prepared by adding fetal calf serum, commercialized by GIBCO Bethesda Research Laboratories, Bethesda, Maryland, USA, to the serum-free culture medium shown in Table 1 below to give a final concentration of 10% (v/v), a final cell density of 1-2 x 10⁴ cells/well, and a final volume of 200 µl; and incubated at 37°C for 48 hours in a 5% CO₂ (v/v) incubator. After completion of the culture, the resultant cells were washed thrice (200 µl x 3) with the serum-free D-MEM medium in Table 1. For a control group, using a fresh preparation of the same medium as used in the above, the saccharide solutions of Nos. 1 to 7 with the compositions shown in Table 2 were prepared, where the saccharide solution of No. 1 consisted of 1 mg/ml of D-glucose with a purity of at least 99%, d.s.b., commercialized by Katayama Chemical Industries, Co., Ltd., Tokyo, Japan; the saccharide solution of No. 2, 1 mg/ml of sucrose with a purity of at least 99%, d.s.b., commercialized by Wako Pure Chemical Industries, Ltd. Tokyo, Japan; the saccharide solution of No. 3, 1 mg/ml of "MALTOSE HHH™", with a purity of at least 99%, d.s.b., commercialized by Hayashibara Biochemical Laboratories, Inc., Okayama, Japan; the saccharide solutions of No. 4, No. 5, and No. 6, which were respectively 1 mg/ml, 0.2 mg/ml, and 0.04 mg/ml of α,α-trehalose with a purity of at least 99%, d.s.b., commercialized by Wako Pure Chemical Industries, Ltd., Tokyo, Japan; and the saccharide solution of No. 7, 1 mg/ml of D-glucose with a purity of at least 99%, d-s.b., commercialized by Wako Pure Chemical Industries, Ltd., Tokyo, Japan. Two hundred microliters of each of the saccharide solutions of Nos.1 to 7 were placed in each well of the 96-well plates with the cells, and the cells were incubated at 37° C for two days in a 5% CO₂ (v/v) incubator. The culture supernatant in each well was sampled and assayed for the production level of OPG by the Western Blotting method as indicated below using a recombinant human OPG commercialized by R & D Systems Inc., MN, USA. In parallel, a cell culture system with no saccharide, as a control group, was provided and cultured, and the supernatant of the cell culture was sampled on day 2 after the initiation of the cell culture and assayed for the production level of OPG similarly as in the test group.

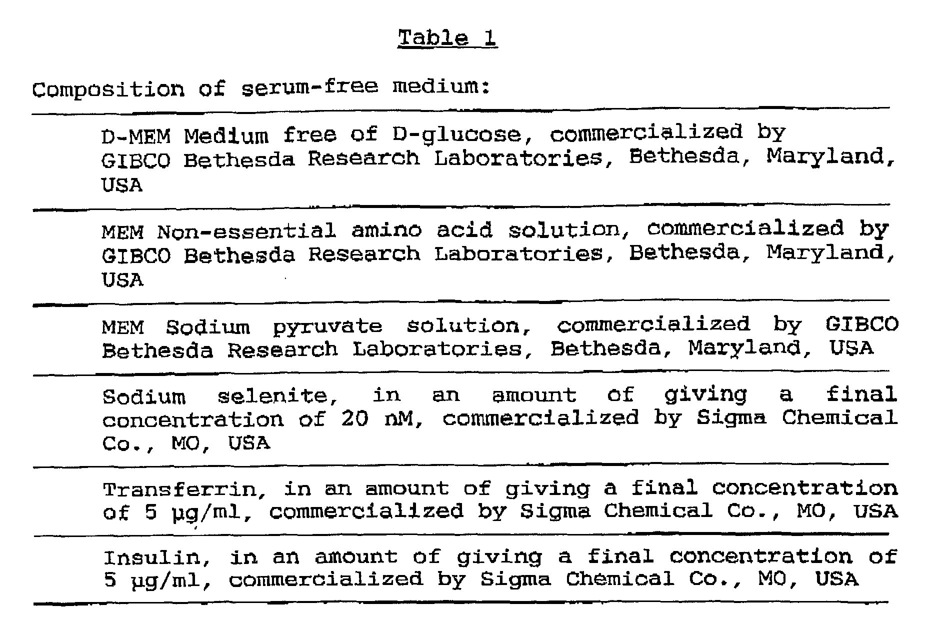

**Table 2**

| Saccharide solution No. | Saccharide | Concentration |
|---|---|---|
| 1 | D-Glucose | 1 mg/ml |
| 2 | Sucrose | 1 mg/ml |
| 3 | Maltose | 1 mg/ml |
| 4 | α,α-Trehalose | 1 mg/ml |
| 5 | α,α-Trehalose | 0.2 mg/ml |
| 6 | α,α-Trehalose | 0.04 mg/ml |
| 7 | D-Glucose + α,α-Trehalose | 1 mg/ml 1 mg/ml |

### Experiment 1-2: Western blotting method

Fifteen microliters of any of the cell culture supernatants of the test solutions and the control solution, and five microliters of dithiothreitol (DTT) solution for cell treatment were mixed and heated at 99°C for five minutes. Each of the resulting solutions with heat treatment was developed on SDS-polyacrylamide gel electrophoresis (SDS-PAGE) using multi-gel 4/20 commercialized by Daiichi Pure Chemicals Co., Ltd., Tokyo, Japan; and the gels were transferred to PVDF membranes commercialized by Nihon Millipore Corporation, Tokyo, Japan. The resulting PVDF membranes were blocked with a solution of "BLOCK ACE™" , commercialized by Dainippon Pharmaceutical Co., Ltd., Tokyo, Japan, and contacted with an anti-human OPG antibody as a primary antibody, commercialized by R & D Systems, MN, USA, and an HRP anti-goat IgG antibody, as a secondary antibody, commercialized by Sigma Chemical Co., MO, USA; followed by detecting proteins reacted with these antibodies in accordance with the method described in Experimental Medicine, special edition, titled Handbook for novel recombinant technology, pp. 222-226 (1999). The coloration was effected by using ECL reagent, commercialized by Amersham Pharmacia Biotechnology, IL, USA; exposing "HYPERFILM ECL™"; and measuring the expression intensity by using IMAGE MASTER™" , commercialized by Amersham Biosciences, Tokyo, Japan. The results are in FIG. 1, where the production inhibitory level of OPG by saccharides is expressed with a relative value (%) by regarding the expression intensity of OPG for the control cell culture system as 100. As a result, when α,α-trehalose was used alone or in combination with D-glucose, the expression intensity of OPG for the cells treated with such saccharides was higher than that for the control cell culture system, while the cells treated with D-glucose, maltose, and sucrose, the expression level of OPG for the cells was lower than that for the control cell culture system. From these results, α,α-trehalose has an action of promoting the production of OPG, while D-glucose, sucrose, and maltose have an action of inhibiting the production of OPG. When α,α-trehalose and D-glucose were used in combination, D-glucose improved the action of α,α-trehalose to enhance the production of OPG.

### Experiment 2: Method for screening substances capable of controlling the production of OPG

Similarly as in Experiment 1 except for using MG-63 cells, ATCC CRL 1427, in place of FHs 74 Int cells, ATCC CCL 241, used in Experiment 1, the saccharide solutions of Nos. 1 to 7 with the compositions in Table 2 were tested whether these saccharides control the production of OPG in MG-63 cells.

As a result, α,α-trehalose distinctly enhanced the production of OPG in the cells compared with a control culture system free of saccharide, revealing that α,α-trehalose is a substance capable of distinctly enhancing the production of OPG. Comparing with the control system free of saccharide, D-glucose, maltose, and sucrose remarkably inhibited the production of OPG, revealing that these saccharides are substances capable of distinctly inhibiting the production of OPG.

Although this experiment exemplified a method for screening different saccharides whether they have an ability of controlling the production of OPG, such a method would be applicable to other natural and synthetic substances similarly as the above-identified saccharides, such as proteins, organic/inorganic compounds, extracts from animal and plant origins, polysaccharides, hormones, and physiologically active substances to examine whether they have an ability of controlling the production of OPG.

### Experiment 3: Acute toxicity test

In a physiological saline containing 5% gum arabic were dissolved α,α-trehalose with a purity of at least 98%, d.s.b., commercialized by Hayashibara Shoji, Inc., Okayama, Japan, and an adequate amount of any of a special reagent grade of D-glucose, maltose, or sucrose with a purity of at least 99%, d.s.b., and the resulting solutions were sterilized in a usual manner. Either of the sterilized saccharide solutions was administered with a syringe or intubationally administered with a stomach sonde to five groups of ddy mice, having 20 to 25 g body weight, consisting of five heads in each group. The mice were observed their conditions for seven days. As a result, no mouse died even when administered with 15 g/kg body weight of α,α-trehalose, D-glucose, maltose, or sucrose, independently of their administration routes. The result indicates that the agent of the present invention is safe even when administered to humans and other mammals.

### Experiment 4: Administration test of agent for controlling the production of OPG

Eighteen healthy volunteers, aged 35 to 50-years-old, were divided into three groups A, B, and C, consisting of three males and three females in each group. Test solution 1 was prepared by dissolving "TREHA®", a crystalline α,α-trehalose with a trehalose content of at least 98%, d.s.b., commercialized by Hayashibara Shoji, Inc., Okayama, Japan, in 1,000 ml deionized water to give a concentration of 10% (w/v), and sterilized with a membrane filter. Test solution 2 was prepared by dissolving D-glucose with a purity of at least 99%, d.s.b., commercialized by Wako Pure Chemical Industries, Ltd., Tokyo, Japan, in 1,000 ml of deionized water to give a concentration of 10% (w/v) and sterilized with a membrane filter. As a control solution, 1,000 ml of deionized water were sterilized with a membrane filter. These test and control solutions were stored at about 10° C in a refrigerator until initiating the test. The volunteers in the groups A to C were allowed to fast, except for water, from 10 p.m. at night just before initiating the test, and 100 ml aliquots of the test solution 1, the test solution 2, and the control solution were administered to the groups A, B, and C, respectively, at 6 a.m. on the next day. Thereafter, all the volunteers were allowed to stay indoors calmly and sampled their blood at six hours after the administrations. The sampled bloods were centrifuged at 35,000 rpm for 10 min and quantified for OPG with "RS000805", a product code number for ELISA system for assaying OPG commercialized by Funakoshi Co., Ltd., Tokyo. Japan. As a result, the production levels of OPG of the volunteers in the test group A, who were orally administered with α,α-trehalose as the test solution 1, were significantly higher than those with the test solution 2 and the control solution. The group B with D-glucose showed a significantly lower production level of OPG than that of the group C with the control solution.

After completion of the administration test, no volunteer, in the group A with **α**,α-trehalose and the group B with D-glucose, complained about abnormality. The data of this experiment revealed that, when orally administered to living bodies, α,α-trehalose enhances the production of OPG but D-glucose inhibits the production thereof.

The preferred embodiments according to the present invention are explained with reference to the following examples:

### Example 1: Health food

According to conventional manner, potatoes, which had been preserved at 20°C and a relative humidity of 85% for two weeks to self-assimilate reducing sugars, were washed with water, pealed, selected, and cut with a slicer into slices, 1.5 mm in thickness. After the slices were washed with water to remove starch on their surfaces, drained water, and fried with a salad oil at 170°C for about five minutes, followed by removing the salad oil. Using a salter, the fried products were uniformly sprayed with a powdery seasoning comprising seven parts by weight of salt, three parts by weight of "TREHA®", a crystalline α,α-trehalose with a trehalose content of at least 98%, d.s.b., commercialized by Hayashibara Shoji, Inc., Okayama, Japan, and an adequate amount of a spice. The resulting products were transferred to a device for weighing and packaging, weighed and packaged with the device, and sealed to obtain a snack-type food.

The product has a satisfactory flavor and taste, and it can be advantageously used as a health food which acts on epithelial cells of mammalian small intestinal mucosae, controls the production of OPG, effectively inhibits the formation of osteoclast relating to osteopathy, and maintains/promotes the health of bone, when orally administered to mammals.

### Example 2: Health food

To 25 parts by weight of well kneaded butter were added 18 parts by weight of "TREHA®", a crystalline α,α-trehalose with a trehalose content of at least 98%, d.s.b., commercialized by Hayashibara Shoji, Inc., Okayama, and 10 parts by weight of chicken eggs in this order, followed by mixing to obtain a creamy product. Forty-seven parts by weight of soft flour were added to the creamy product, and the resulting mixture was kneaded and wrapped with a cloth and allowed to stand for 20 min. The resulting dough was shaped into a rod form, 3 cm in diameter, wrapped with a paraffin paper, and allowed to stand at 4°C for two hours. The resulting rod-shaped dough was sliced in rounds, 5 mm in thickness, and the slices were placed on a plate with grease, baked in an oven at 170°C for 10 min, applied with "TREHASTAR®", a trehalose syrup with an α,α-trehalose content of at least 28%, d.s.b., commercialized by Hayashibara Shoji, Inc., Okayama, Japan, and further baked at the same temperature as in the above for 10 min to obtain an icebox-type cookie.

The product has a satisfactory flavor and taste, and it can be advantageously used as a health food that acts on epithelial cells of mammalian small intestinal mucosae, controls the production of OPG, effectively inhibits the formation of osteoclast relating to osteopathy, and maintains/promotes the health of bone, when orally administered to mammals.

### Example 3: Health food

Seven parts by weight of a freeze-dried tea extract, three parts by weight of α,β-trehalose, and three parts by weight of D-glucose were dissolved in water. The resulting solution was sprayed over 90 parts by weight of tea leaves which had been fermented and dried in a usual manner. According to conventional manner, the tea leaves thus obtained were sieved, cut, dried for finish, subjected to a separator for removing impurities, and packed with a Japanese paper in an amount of two grams.

Before drinking, the product is soaked for extraction in 180 ml of cold water for about 10 min or of hot water heated to 90 to 100°C for about two min.

The product has a satisfactory flavor and taste, and it can be advantageously used as a health food that acts on epithelial cells of mammalian small intestinal mucosae, controls the production of OPG, effectively inhibits the formation of osteoclast relating to osteopathy, and maintains/promotes the health of bone, when orally administered to mammals.

### Example 4: Health food

A coffee-type beverage was prepared by mixing in a usual manner 2.7 parts by weight of "TETRUP®", a maltotetraose syrup containing, on a dry solid basis, about 2% glucose, about 7% maltose, about 11% maltotriose, about 50% maltotetraose, and about 28% dextrin, commercialized by Hayashibara Shoji, Inc., Okayama, Japan; seven parts by weight of "TREHASTAR®", a trehalose syrup with an α,α-trehalose content of at least 28%, d.s.b., commercialized by Hayashibara Shoji, Inc., Okayama, Japan; five parts by weight of a coffee extract, 2.2 parts by weight of a whole milk powder, one part by weight of a skim milk powder, 0.04 part by weight of sucrose fatty acid ester, 0.06 part by weight of sodium bicarbonate, and 82 parts by weight of water.

The product has a satisfactory flavor and taste, and it can be advantageously used as a health food that acts on epithelial cells of mammalian small intestinal mucosae, controls the production of OPG, effectively inhibits the formation of osteoclast relating to osteopathy, and maintains/promotes the health of bone, when orally administered to mammals.

### Example 5: Health food

Fifty-five parts by weight of "TREHA®", a crystalline α,α-trehalose powder with an α,α-trehalose content of at least 98%, d.s.b., commercialized by Hayashibara Shoji, Inc., Okayama, 40.5 parts by weight of corn starch, and 2.5 parts by weight of crystalline cellulose were mixed and then in a usual manner kneaded while spraying and dropping thereto a small amount of water, subjected to fluidized-bed granulation, pulverized, and sized to obtain a powder for tabletting. To the powder was added two parts by weight of sucrose fatty acid ester, and the mixture was mixed to homogeneity and tabletted by a tabletting machine with a punch of 11 mm to obtain a tablet containing α,α-trehalose, about 300 mg each.

The product has a satisfactory swallowability and degradability in intestines, and it can be advantageously used as a health food that acts on epithelial cells of mammalian small intestinal mucosae, controls the production of OPG, effectively inhibits the formation of osteoclast relating to osteopathy, and maintains/promotes the health of bone, when orally administered to mammals.

### Example 6: Health supplement

Thirty-nine parts by weight of "TREHA®", a crystalline α,α-trehalose powder with an α,α-trehalose content of at least 98%, d.s.b., commercialized by Hayashibara Shoji, Inc., Okayama, 25 parts by weight of a natural coral powder, 12 parts by weight of a yogurt powder, 10 parts by weight of guar gum, 1.9 parts by weight of 2-0-α-D-glucopyranosyl-L-ascorbic acid, and 0.1 part by weight of α-glycosyl hesperidin were in a usual manner kneaded while spraying and dropping to the contents an adequate amount of water, subjected to fluidized-bed granulation, pulverized, and sized to obtain a powder for tabletting. After homogeneously mixing the powder with three parts by weight of sucrose fatty acid ester as a gloss-imparting agent, the resulting mixture was tabletted by a tabletting machine with a punch of 6 mm in diameter to obtain tablets, about 200 mg each.

The product is enriched with calcium and easily taken orally, and it can be advantageously used as a health food that acts on epithelial cells of mammalian small intestinal mucosae, controls the production of OPG, effectively inhibits the formation of osteoclast relating to osteopathy, and maintains/promotes the health of bone, when orally administered to mammals.

### Example 7: Health supplement

Three parts by weight of "TREHA®" , a crystalline α,α-trehalose powder with an α,α-trehalose content of at least 98%, d.s.b., commercialized by Hayashibara Shoji, Inc., Okayama, one part by weight of α,β-trehalose with a purity of at least 95%, d.s.b., one part by weight of β,β-trehalose, and three parts by weight of pullulan were tabletted with a tabletting machine with a punch of 6 mm in diameter to obtain tablets, about 300 mg each.

The product has an adequate strength because of its pullulan, and it can be advantageously used as a health food that acts on epithelial cells of mammalian small intestinal mucosae, controls the production of OPG, effectively inhibits the formation of osteoclast relating to osteopathy, and maintains/promotes the health of bone, when orally administered to mammals.

### Industrial Applicability

As explained above, the agent for controlling the production of OPG, which comprises a saccharide as an effective ingredient, has an advantageous feature that effectively controls the formation of osteoclast. Since the agent comprises a saccharide(s) as an effective ingredient, it has a satisfactory flavor and taste, and it can be easily taken or administered to living bodies without causing any inconvenience through oral and incubational administration routes, moderately acts on epithelial cells of mammalian small intestinal mucosae, and controls the production of OPG in the cells. As a result, the agent of the present invention can be advantageously used to effectively control the formation of osteoclast, which closely relates to bone diseases, and used to maintain/promote the health of bone.

The method for screening substances capable of controlling the production of OPG according to the present invention enables to screen potential substances to be used in the agent of the present invention as effective ingredients extensively, promptly, and easily.

The present invention with such outstanding functions and effects is a significant invention that greatly contributes to this art.

## Claims

1. An agent for controlling the production of osteoprotegerin, which comprises a saccharide as an effective ingredient.

2. The agent of claim 1, wherein said saccharide is one or more members selected form the group consisting of D-glucose, maltose, sucrose, α,α-trehalose, α,β-trehalose, and β,β-trehalose.

3. The agent of claim 1 or 2, which further contains a calcium preparation.

4. The agent of claim 1, 2 or 3, which acts on epithelial cells of mammalian small intestinal mucosae and controls the production of osteoprotegerin in the cells.

5. The agent of any one of claims 1 to 4, which is in an orally or intubationally administrable form.

6. A method for screening a substance capable of controlling the production of osteoprotegerin, which comprises the steps of:
culturing an established cell line capable of producing osteoprotegerin in a nutrient culture medium;
adding a test sample to the cell culture;
culturing the cells in the presence of the test sample to produce osteoprotegerin;
assaying the production level of osteoprotegerin in the culture supernatant; and
judging the test sample to be a positive substance that can control the production of osteoprotegerin when the production level of osteoprotegerin for the test sample is higher or lower than that for a control system.

7. The method of claim 6, wherein said established cell line is MG-63 cells, ATCC CRL 1427; or FHs 74 Int cells, ATCC CCL 241.

8. An agent for controlling the production of osteoprotegerin, which comprises as an effective ingredient a substance capable of controlling the production of osteoprotegerin, said substance having been screened by the method of claim 6 or 7.
